# EUROPÄISCHE PATENTSCHRIFT

(11) **EP 0 997 456 B1**
(45) Veröffentlichungstag und Bekanntmachung des Hinweises auf die Patenterteilung: **26.02.2003**
(21) Anmeldenummer: 99119594.2
(22) Anmeldetag: 02.10.1999
(51) Int. Cl.: C07C 407/00, C07C 409/10

(54) **Verfahren zur Rückgewinnung von Cumolhydroperoxid aus hydroperoxidhaltigem Wasser**
Process for recycling cumene hydroperoxide from hydroperoxide containing water
Procédé de récupération de l'hydropéroxyde de cumène de l'eau contenant d'hydropéroxydes

(30) Priorität: 09.10.1998 DE 19846508
(43) Veröffentlichungstag der Anmeldung: 03.05.2000
(73) Patentinhaber: Domo Caproleuna GmbH, 06237 Leuna (DE)
(72) Erfinder: Hofmann, Günter, Dipl.-Ing., 06124 Halle (DE); Pester, Rolf, Dr., 06231 Bad Dürrenberg (DE); Bartkowiak, Horst, Dipl.-Ing., 06237 Leuna (DE)
(74) Vertreter: Schinke, Herbert, Dr. Dr., Patentanwaltskanzlei

(56) Entgegenhaltungen:
- US-A- 4 120 902
- CHEMICAL ABSTRACTS, vol. 79, no. 15, 15. Oktober 1973 (1973-10-15) Columbus, Ohio, US; abstract no. 91775, KATO, MITSUKUNI: "Separating cemene hydroperoxide from cumene by extraction with glycols" XP002129480 & JP 48 023069 B (NIPPON OILS AND FATS CO., LTD.) 11. Juli 1973 (1973-07-11)

## Beschreibung

Die Erfindung betrifft ein Verfahren zur Rückgewinnung von Cumolhydroperoxid aus hydroperoxidhaltigem Wasser, insbesondere zur Behandlung von hydroperoxidhaltigem Abwasser, das bei der Phenolherstellung auf Basis Cumol anfällt. Die Hydroperoxid-Bestandteile des Abwassers sind vor allem Methylhydroperoxid und Cumolhydroperoxid.

Cumolhydroperoxid ist darüber hinaus ein Zwischenprodukt der Phenolherstellung:

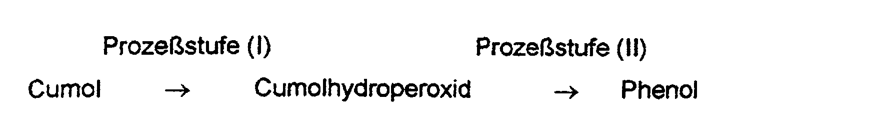

Das Abwasser fällt in der Prozeßstufe (I) an.

Es ist bekannt, daß Hydroperoxide den biologischen Abbau, speziell die Nitrifizierung und Denitrifizierung, inhibieren und somit die Behandlung solcher Abwässer in biologischen Reinigungsanlagen problematisch ist.

Es ist ferner bekannt, daß Phenolproduzenten dieses Abwasser erst nach Vermischung mit größeren Mengen anderer Abwässer einer biologischen Abwasserbehandlung zuführen und somit eine solche Verdünnung der Hydroperoxide realisieren, bei der deren inhibierende Wirkung weitgehend reduziert wird.

Diese Verfahrensweise ist jedoch nur dann möglich, wenn ausreichend andere Abwässer zur wirtschaftlichen Verdünnung des hydroperoxidhaltigen Abwassers am Ort der biologischen Abwasserreinigung zur Verfügung stehen.

Auf diese Weise gehen jedoch die im Abwasser enthaltenen Wertstoffe wie Cumolhydroperoxid und Dimethylphenylcarbinol verloren.

Es bestand deshalb die Aufgabe, diese zu gewinnen und für die Phenolherstellung nutzbar zu machen. Dazu ist ihre Abtrennung vom Methylhydroperoxid notwendig.

Verfahren zur Extraktion von Cumolhydroperoxid werden in US-A-4 120 902 sowie in JP-B-48 023 069 genannt.

Es wurde gefunden, daß die Rückgewinnung von Cumolhydroperoxid aus hydroperoxidhaltigem Wasser dadurch gelingt, daß dieses mit Cumol extrahiert wird. Die Extraktphase enthält fast die gesamte Menge dieser Stoffe und nur Spuren von Methylhydroperoxid. Das Methylhydroperoxid und andere den Cumoloxidationsprozeß (Prozeßstufe 1) störende Inhaltsstoffe wie Ameisensäure, Methanol und Phenolspuren verbleiben in der wäßrigen Phase.

Besonders vorteilhaft ist es natürlich, wenn das Cumol verwendet wird, das bei der industriellen Phenolherstellung zum Einsatz kommt oder gebildet wird.

Dabei kann man für den Extraktionsprozeß die insgesamt für den Produktionsprozeß benötigte Cumolmenge oder eine Teilmenge einsetzen. Bevorzugt wird der Einsatz der Gesamtmenge.

Das bedeutet, daß man für den Extraktionsprozeß vorzugsweise 1 Teil bis 8 Teile Cumol, vorzugsweise 4 bis 5 Teile Cumol, pro 1 Teil Abwasser einsetzt.

Es hat sich als zweckmäßig erwiesen, wenn der pH-Wert des zu extrahierenden Wassers im Bereich 7 bis 12, vorzugsweise bei 7,5 bis 9, liegt.

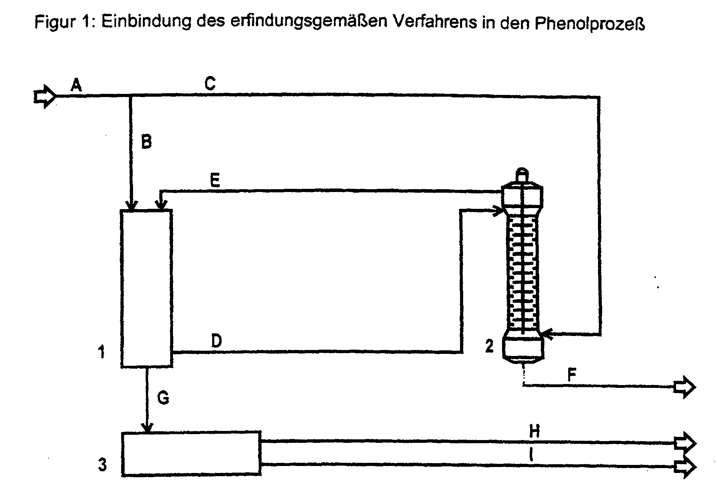

Das Verfahren soll am Fließschema gemäß Figur 1 erläutert werden.

Darin bedeuten
- 1: Prozeßstufe 1
- 2: Extraktor
- 3: Prozeßstufe 2
- A: Cumol Gesamtmenge
- B: Cumol Teilmenge
- C: Cumol Teilmenge
- D: Hydroperoxidhaltiges Abwasser
- E: Extraktcumol
- F: cumolhydroperoxidfreies Abwasser
- G: Cumolhydroperoxid
- H: Phenol
- l: Aceton

Die gesamte für den Phenolprozeß benötigte Cumolmenge (A) wird entweder vollständig in die erfindungsgemäße Extraktion in den Extraktor (2) eingespeist oder in die Teilmengen (B) und (C) aufgesplittet. Davon wird eine Teilmenge (B) direkt in die Prozeßstufe 1(1) und nur die Teilmenge (C) zur Extraktion verwendet.

Im Extraktor (2) wird das hydroperoxidhaltige Abwasser (D) aus der Prozeßstufe 1 (1) mit dem Cumolstrom (C) im Gegenstrom extrahiert. Das am Kopf des Extraktors (2) anfallende Extraktcumen enthält das aus der wäßrigen Phase extrahierte Cumolhydroperoxid und andere Wertstoffe und wird der Prozeßstufe 1(1) zugeführt.

Das am Boden des Extraktors (2) anfallende cumolhydroperoxidfreie Abwasser (F) kann dann in einer Nachbehandlungsstufe auf chemischer und/oder biologischer Basis von den restlichen organischen Inhaltsstoffen befreit und nachfolgend als gereinigtes Abwasser in ein Fließgewässer abgeleitet werden.

Das Cumolhydroperoxid (G) wird in der Prozeßstufe 2 (3) zu den Endprodukten Phenol (H) und Aceton (I) aufgearbeitet.

Durch das erfindungsgemäße Verfahren können mehr als 99 % des im Abwasser enthaltenen Cumolhydroperoxides und Dimethylphenylcarbinols zurückgewonnen werden. Außerdem verringert sich dadurch der chemische Sauerstoffbedarf des Abwassers (CSB) um ca. 35 %, verbunden mit einer deutlichen Reduzierung der Kosten für die nachfolgende biologische Abwasserbehandlung.

Das nach der erfindungsgemäßen Behandlung anfallende Abwasser enthält praktisch das gesamte Methylhydroperoxid und nur Spuren von Cumolhydroperoxid.

Zur Verbesserung der biologischen Abbaubarkeit kann das Methylhydroperoxid durch Reaktion mit Reduktionsmitteln zerstört werden, was jedoch mit erheblichem Aufwand an Chemikalien und Kosten verbunden ist.

Zweckmäßig ergänzt man deshalb das erfindungsgemäße Abwasserbehandlungsverfahren mit einem bereits bekannten Verfahren, vorzugsweise mit dem in der DE-Anmeldung 198 24 076 vorgeschlagenen Verfahren zur katalytischen Reinigung peroxidhaltiger Abwässer. Dabei werden mindestens 99 % des Methylhydroperoxides in hinsichtlich der biologischen Abbaubarkeit unproblematische Stoffe umgewandelt, und das Abwasser kann ohne vorhergehende Verdünnung einer normalen biologischen Behandlungsanlage zur Eliminierung der restlichen organischen Stoffe zugeführt werden.

### Beispiel 1

Aus einem Vorratsbehälter werden gemäß Spalte 1 der Tabelle kontinuierlich stündlich 5000 kg 30 °C warmes Abwasser mit 4100 mg/l Cumolhydroperoxid, 16200 mg/l Methylhydroperoxid, 7100 mg/l Ameisensäure (als Formiat), 17 mg/l Phenol (als Phenolat), 227 mg/l Dimethyl-phenylcarbinol, 642 mg/l Methanol, 193 mg/l Aceton, 20 mg/l Cumol, einem CSB-Wert von 12200 mgO₂/l und einem pH-Wert von 8,5 in den oberen Teil eines Drehscheibenextraktors mit drei theoretischen Trennstufen eingespeist. 22500 kg cumolhydroperoxidfreies Cumol (im weiteren als Reincumol bezeichnet) pro Stunde als Extraktionsmittel werden in den unteren Teil des Apparates eingeleitet, mit dem entgegenströmenden Abwasser intensiv gemischt und nach einer Phasentrennung am Kopfteil des Extraktors als Extrakt abgezogen.

Das am Sumpf des Extraktors als Raffinat anfallende behandelte Abwasser ist praktisch frei von Cumolhydroperoxid und Dimethylphenylcarbinol, besitzt einen CSB-Wert von nur noch 7800 mg O₂/l (s. Spalte 1 der Tabelle) und wird zum katalytischen Abbau des Methylhydroperoxids einer Abwasserbehandlungsanlage danach und nachfolgend einer biologischen Reinigung zugeführt.

### Beispiel 2

1000 kg Abwasser pro Stunde mit einer Zusammensetzung gemäß Spalte 2 der. Tabelle werden in einem Vibrationsextraktor mit 5 theoretischen Trennstufen im Gegenstrom mit stündlich 1000 kg Reincumol extrahiert. Im behandelten Abwasser ist kein Cumolhydroperoxid und Dimethylphenylcarbinol nachweisbar und der CSB-Wert ist um 30 % von 15600 mg O₂/l im unbehandelten Abwasser auf 11050 mg O₂/1 im behandelten Abwasser reduziert (s. Spalte 2 der Tabelle).

### Beispiel 3

In einer Mixer-Settler-Einheit, bestehend aus einem statischen Mischer, einem Trennapparat und den zugehörigen Pumpen und Armaturen, werden stündlich 700 kg Abwasser mit einer Zusammensetzung gemäß Spalte 3 der Tabelle mit 5200 kg Reincumol pro Stunde intensiv gemischt und nachfolgend in eine Extrakt- und eine Raffinatphase getrennt. Dabei wird der Cumolhydroperoxidgehalt des Abwassers um 98 % von 919 mg/l auf 16 mg/l abgesenkt (s. Spalte 3 der Tabelle). Nach Zersetzung des Methylhydroperoxids kann das Abwasser einer biologischen Reinigungsstufe zugeführt werden.

### Beispiel 4

Stündlich 5200 kg Abwasser mit einer Zusammensetzung gemäß Spalte 4 der Tabelle werden in einer Packungskolonne mit drei theoretischen Trennstufen im Gegenstrom mit 31000 kg Reincumol extrahiert. Es wird ein behandeltes Abwasser mit Parametern gemäß Spalte 4 der Tabelle erhalten, das entsprechend Beispiel 1 weiterverarbeitet werden kann.

Tabelle zu den Beispielen 1 bis 4

| **Beispiel** | | **1** | **2** | **3** | **4** |
|---|---|---|---|---|---|
| Extraktortyp | | Drehscheiben- extraktor | Vibrattons- extraktor | Mixer- Settler | Packungskolonne |
| theoretische Trennstufenzahl | | 3 | 5 | 1 | 3 |
| **Abwasser vor Behandlung:** | | | | | |
| Menge | kg/h | 5000 | 1000 | 700 | 5200 |
| Temperatur | °C | 30 | 40 | 24 | 35 |
| p_{H}-Wert | | 8.5 | 12 | 7 | 9 |
| Zusammensetzung: | | | | | |
| Cumol hydroperoxid | mg/l | 4100 | 7970 | 919 | 10240 |
| Methylhydroperoxid | mg/l | 16200 | 13400 | 7150 | 14050 |
| Ameisensäure (als Formiat) | mg/l | 7100 | 9800 | 1200 | 8030 |
| Phenol (als Phenolat) | mg/l | 17 | 35 | 1 | 27 |
| Dimethylphenylcarbinol | mg/l | 227 | 400 | 316 | 600 |
| Methanol | mg/l | 642 | 410 | 513 | 310 |
| Aceton | mg/l | 193 | 310 | 205 | 265 |
| Cumol | mg/l | 20 | 50 | 90 | 70 |
| CSB | mg O₂/I | 12200 | 15600 | 8900 | 17300 |
| **Extraktionsmittel:** | | Cumol | Cumol | Cumol | Cumol |
| Menge | kg/h | 22500 | 1000 | 5200 | 31000 |
| Temperatur | °C | 30 | 32 | 18 | 36 |
| **Abwasser nach Behandlung:** | | | | | |
| Zusammensetzung: | | | | | |
| Cumol hydroperoxid | mg/l | <3 | <3 | 16 | <3 |
| Methylhydroperoxid | mg/l | 16270 | 13450 | 7170 | 14100 |
| Ameisensäure (als Formiat) | mg/l | 7130 | 9840 | 1210 | 8070 |
| Phenol (als Phenolat) | mg/l | 17 | 35 | 1 | 27 |
| Dimethylphenylcarbinol | mg/l | < 3 | <3 | 3 | <3 |
| Methanol | mg/l | 650 | 415 | 516 | 315 |
| Aceton | mg/l | 201 | 320 | 210 | 270 |
| Cumol | mg/I | 50 | 40 | 30 | 35 |
| CSB | mg O₂/l | 7800 | 10050 | 5700 | 11400 |

## Patentansprüche

1. Verfahren zur Rückgewinnung von Cumolhydroperoxid aus hydroperoxidhaltigem Wasser, **dadurch gekennzeichnet, daß** dieses mit Cumol extrahiert wird.

2. Verfahren nach Anspruch 1, **dadurch gekennzeichnet, daß** Cumol verwendet wird, das bei der industriellen Phenolherstellung zum Einsatz kommt oder gebildet wird.

3. Verfahren nach Anspruch 1 und 2, **dadurch gekennzeichnet, daß** man für den Extraktionsprozeß die insgesamt für den Produktionsprozeß benötigte Cumolmenge einsetzt.

4. Verfahren nach einem oder mehreren der Ansprüche 1 bis 3, **dadurch gekennzeichnet, daß** man für den Extraktionsprozeß 1 Teil bis 8 Teile Cumol pro 1 Teil Abwasser einsetzt.

5. Verfahren nach einem oder mehreren der Ansprüche 1 bis 4, **dadurch gekennzeichnet, daß** man für den Extraktionsprozeß 4 bis 5 Teile Cumol pro 1 Teil Abwasser einsetzt.

6. Verfahren nach einem oder mehreren der Ansprüche 1 bis 4, **dadurch gekennzeichnet, daß** der pH-Wert des zu extrahierenden Wassers im Bereich 7 bis 12 liegt.

7. Verfahren nach einem oder mehreren der Ansprüche 1 bis 6, **dadurch gekennzeichnet, daß** der pH-Wert des zu extrahierenden Wassers im Bereich 7,5 bis 9 liegt.

## Claims

1. A method for recovering cumol hydroperoxide from hydroperoxide-containing water wherein said water is extracted with cumol.

2. The method according to claim 1 wherein the cumol used is applied or formed in the industrial-scale production of phenol.

3. The method according to claims 1 and 2 wherein the total cumol quantity required for the production process is used in the extraction process.

4. The method according to one or several of claims 1 through 3 wherein 1 part to 8 parts of cumol per 1 part of wastewater are used for the extraction process.

5. The method according to one or several of claims 1 through 4 wherein 4 to 5 parts of cumol per 1 part of wastewater are used for the extraction process.

6. The method according to one or several of claims 1 through 4 wherein the pH value of the water to be extracted is in the range from 7 to 12.

7. The method according to one or several of claims 1 through 6 wherein the pH value of the water to be extracted is in the range from 7.5 to 9.

## Revendications

1. Procédé de récupération de l'hydropéroxyde de cumène à partir d'eau contenant de l'hydropéroxyde, **caractérisé en ce que** celle-ci est extraite avec du cumène.

2. Procédé selon la revendication 1, **caractérisé en ce qu'**on utilise du cumène qui est mis en oeuvre ou formé lors de la production industrielle de phénol.

3. Procédé selon l'une ou l'autre des revendications 1 et 2, **caractérisé en ce qu'**on utilise pour le processus d'extraction la quantité de cumène nécessaire dans l'ensemble pour le processus de production.

4. Procédé selon l'une ou plusieurs des revendications 1 à 3, **caractérisé en ce qu'**on utilise pour le processus d'extraction 1 à 8 parts de cumène pour 1 part d'eaux usées.

5. Procédé selon l'une ou plusieurs des revendications 1 à 4, **caractérisé en ce qu'**on utilise pour le processus d'extraction 4 à 5 parts de cumène pour 1 part d'eaux usées.

6. Procédé selon l'une ou plusieurs des revendications 1 à 4, **caractérisé en ce que** la valeur de pH de l'eau à extraire est située dans la plage de 7 à 12.

7. Procédé selon l'une ou plusieurs des revendications 1 à 6, **caractérisé en ce que** la valeur de pH de l'eau à extraire est située dans la plage de 7,5 à 9.
